# EUROPEAN PATENT SPECIFICATION

(11) **EP 3 620 136 B1**
(45) Date of publication and mention of the grant of the patent: **14.04.2021**
(21) Application number: 19196086.3
(22) Date of filing: 09.09.2019
(51) Int. Cl.: A61F 5/058, A61F 5/01, A61F 5/05, A61F 5/24

(54) **WRIST ORTHOSIS**
HANDGELENKORTHESE
ORTHÈSE DE POIGNET

(30) Priority: 10.09.2018 NL 2021587
(43) Date of publication of application: 11.03.2020
(73) Proprietor: WE Design Beheer B.V., 6814 EN Arnhem (NL)
(72) Inventor: ENGELSHOVEN, Wouter Robin, 6815 AK Arnhem (NL)
(74) Representative: Nederlandsch Octrooibureau

(56) References cited:
- WO-A1-2014/070625
- US-A- 4 854 310
- US-A- 5 695 453

## Description

### Field of the invention

The present invention relates to an orthopaedic orthosis, in particular a frame/wire-based wrist orthosis for immobilizing the wrist of a patient.

### Background art

WO2014070625 discloses a "bikini brace" to immobilise a wrist comprising a distal hand portion, a middle section that includes an upper elongated portion and a lower elongated portion that extend along the length of the brace and a proximal forearm portion. The upper and lower portions are configured to extend in upper and lower fashion along the wrist. US5695453 discloses a brace to immobilise a wrist containing an immobilizer comprising a sheet of soft pliant foam material that defines the body of the immobilizer and is formable in encircling relationship to the extremity. The sheet of foam material has embedded therein a system of wire-like reinforcing and stabilizing members that run along the upper and lower side of the wrist. US4854310 discloses a wrist orthosis for supporting the wrist of a patient represented by a splint comprising a sheet for the lower arm that comprises a radial wire and an ulnar wire that are connected by a mesh of several struts. In addition to the sheet, the splint also comprises as a second member a rigid collar in the shape of an elongated letter "C" extending circumferentially halfway around the hand. Said collar is connected to the sheet by screws. There are known wrist orthoses made of a frame/wire which is shaped to snugly fit around a patient's wrist for support thereof. An advantage of such a frame/wire-based wrist orthosis is that it is waterproof and that it provides a low profile and sleek design, allowing the wrist orthosis to not interfere as much with clothing around a patient's arm compared to other known wrist orthoses.

However, even though such a frame-based wrist orthosis may provide the required support from an orthopaedic point of view, the frame may sometimes cause discomfort to a patient. For example, in particular cases a frame-based wrist orthosis may cause discomfort at places where the skin and underlying tissue on bone structure is relatively thin, causing localized pressure points that may not be sufficiently absorbed and diffused by tissue, especially when the thumb and index fingers are pressed together for gripping, thereby tensioning the tendons on the dorsal side of the hand.

### Summary of the invention

The present invention aims to provide an improved wrist orthosis, such as a frame/wire-based wrist orthosis, configured to limit volar and/or dorsal flexion of a patient's hand and as such provides volar and dorsal stabilisation, as described in the claims. Ulnar and radial stabilisation is also provided by the wrist orthosis. The wrist orthosis is further configured to reduce localised pressure points without sacrificing the required orthopaedic support of the wrist.

According to the present invention, a wrist orthosis of the type defined in the claims is provided comprising a wire frame which is configured to a engage a forearm, wrist, and hand of a patient, wherein the wire frame comprises a continuous connection of
a radial wire section configured to extend in a distal direction along the forearm and a radial side of the hand to an interdigital space between the thumb and index finger, wherein the radial wire section is connected to
a palmar wire section configured to extend in a lateral direction from the interdigital space along a palmar region of the hand toward an ulnar side of the hand, and wherein the palmar wire section is connected to
an ulnar wire section configured to extend from the ulnar side of the hand in proximal direction toward and along the forearm; and wherein
the radial wire section and the ulnar wire section are laterally spaced apart along an intermediate dorsal region of the hand.

According to the present invention, the lateral space between the radial wire section and the ulnar wire section along the intermediate dorsal region of the hand effectively provides for more freedom of movement of both the index and middle finger by avoiding any pressure on the dorsal side of the metacarpals and tendons of the hand.

The lateral space between the radial wire section and the ulnar wire section along the intermediate dorsal region further provides an open construction between the radial and ulnar part of the hand allowing adjustment to fit a size/width of the hand by e.g. bending the radial wire section and the ulnar wire section towards or away from each other.

In advantageous embodiments, the wire frame is a metallic or metal alloy wire frame, allowing the wrist orthosis to be bent for achieving a desired orthopaedic shape, but also for making small or minor adjustments by bending the wire frame where needed. In further advantageous embodiments, the wire frame comprises silver, gold, bronze or titanium or a combination thereof, where these material are biologically inert and as such minimize bacterial growth on and around the wrist orthosis.

### Brief description of the drawings

The present invention will now be described by way of reference to a number of illustrative embodiments as shown in the accompanying drawings in which:
Figure 1 shows a dorsal view of a prior art wrist orthosis;
Figure 2 shows a dorsal view of a wrist orthosis according to an embodiment of the present invention;
Figure 3 shows a volar view of a wrist orthosis according to an embodiment of the present invention;
Figure 4 shows a dorsal-ulnar view of a wrist orthosis provided according to an embodiment of the present invention;
Figure 5 shows a dorsal view of a wrist orthosis comprising a thumb brace according to an embodiment of the present invention; and
Figure 6 shows a volar-radial view of a wrist orthosis comprising a thumb brace according to an embodiment of the present invention.

### Detailed description of embodiments.

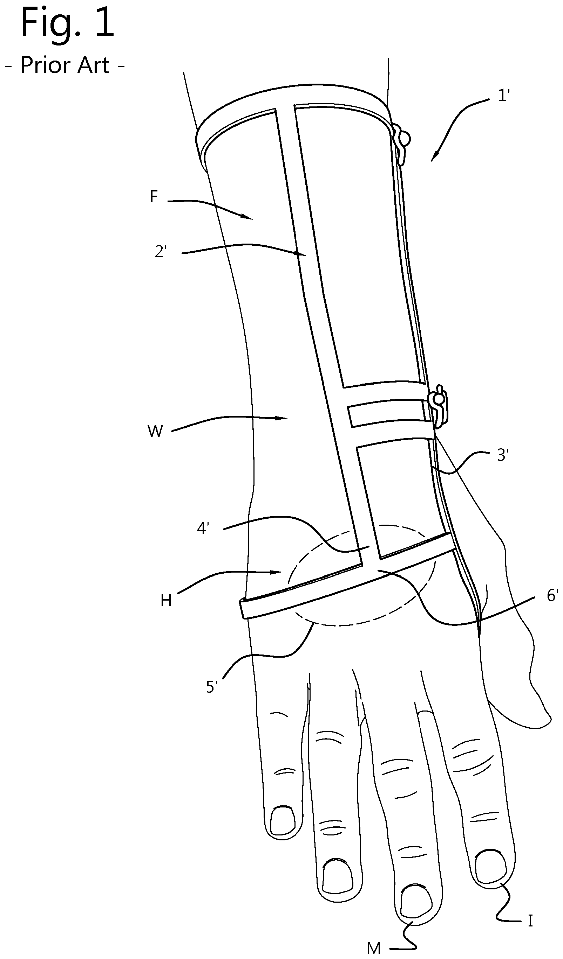

Figure 1 shows a dorsal view of a prior art wrist orthosis 1' as worn by a patient. The wrist orthosis 1' comprises a wire frame 2' configured to engages a forearm F, wrist W, and hand H of a patient. The wire frame 2' comprises a radial wire section 3' and an ulnar wire section 4' that are laterally spaced apart. The radial wires section 3' and the ulnar wire section 4' are connected along an intermediate dorsal region 5' of the hand by means of a laterally arranged dorsal connecting wire section 6'. As shown, the intermediate dorsal region 5' of the hand is approximately located along a dorsal side of the metacarpals associated with the index finger I and middle finger M.

Patients wearing this particular prior art wrist orthosis 1' have indicated that painful pressure points might develop where the dorsal connecting wire section 6' engages the dorsal side of the hand, in particular the intermediate dorsal region 5'. Investigation has shown that dorsal movement/flexion of the index and middle fingers I, M sometimes causes the dorsal side of the hand to firmly press against the dorsal connecting wire section 6' and as a result painful pressure points might develop.

Based on the above observation, there is a need for a wrist orthosis which eliminates painful pressure points along the dorsal side of the hand whilst maintaining required orthopedic support of a patient's wrist, such as sufficient volar and dorsal support as well as radial and ulnar stabilization.

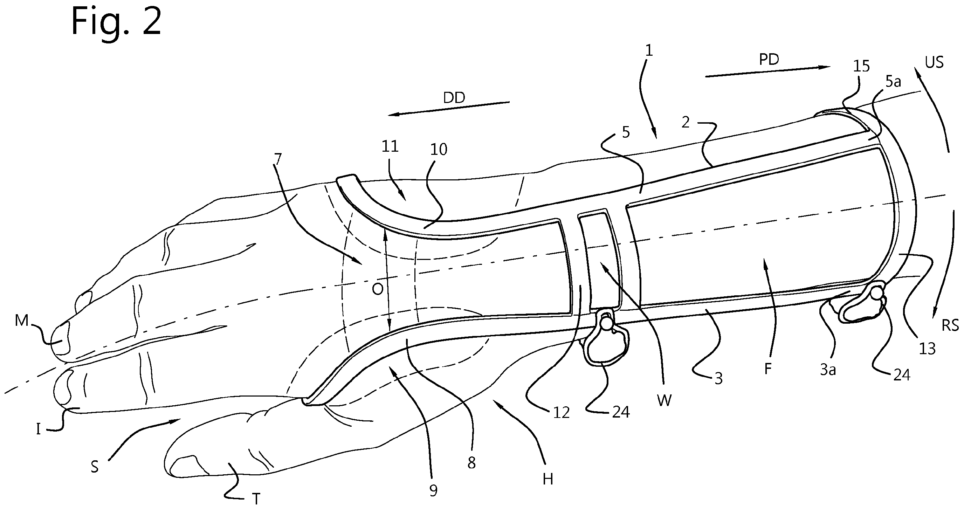

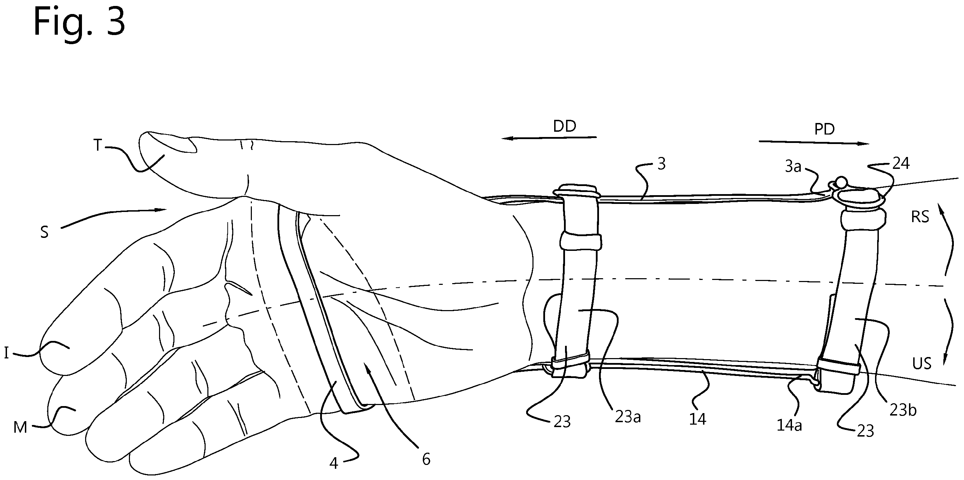

Figure 2 and 3 show a dorsal and volar view, respectively, of a wrist orthosis 1 according to an embodiment of the present invention. In the embodiments shown, the wrist orthosis 1 comprises a wire frame 2 which is configured to a engage a forearm F, wrist W, and hand H of a patient. The wire frame 2 comprises a continuous connection of a radial wire section 3 which is configured to extend in a distal direction, indicate as "DD", along the forearm and a radial side of the hand, see indication "RS", to an interdigital space S between the thumb T and index finger I. The radial wire section 3 further connects to a palmar wire section 4 which is configured to extend in lateral direction from the interdigital space S along a palmar region 6 of the hand toward an ulnar side of the hand, indicated as "US". The palmar wire section 4 is connected to an ulnar wire section 5 which is configured to extend from the ulnar side in proximal direction, indicated as "PD", toward and along the wrist and forearm.

For maximum clarity, the term "lateral" or "laterally" is to be understood as being a direction sideways from the distal direction DD and proximal direction PD.

As further shown in Figure 1, the radial wire section 3 and the ulnar wire section 5 are laterally spaced apart along an intermediate dorsal region 7 of the hand as indicated by a lateral separation distance "O".

According to the present invention, the space between the radial wire section 3 and the ulnar wire section 5 provides for an "open construction" along the intermediate dorsal region 7. Consequently, engagement between the dorsal side of the hand and the wrist orthosis 1 is reduced considerably and as such pressure points are avoided. The open construction indicated by the separation distance O along the intermediate dorsal region 7 is particularly advantageous for increased freedom of (dorsal) movement of the index and middle fingers I, M.

Another advantage of laterally separating the radial wire section 3 and the ulnar wire section 5 along the intermediate dorsal region 7, is that the wire frame 2 exhibits flexibility and as such can be bent to specifically fit a patient's anatomy, such as a width of the hand. For example, the separation distance O between the radial wire section 3 and the ulnar wire section 5 can be adjusted by bending the radial wire section 3 and the ulnar wire section 5 toward or away from each other at the intermediate dorsal region 7.

In an advantageous embodiment, the radial wire section 3, the palmar wire section 4, and the ulnar wire section 5 are formed as a continuous single piece of bent wire, thereby allowing for a smooth, organically shaped wrist orthosis 1 that snugly and comfortably fits to a patients forearm, wrist and hand. In addition, the single piece of bent wire further facilitates adjustment of the separation distance O between the radial wire section 3 and the ulnar wire section 5 along the intermediate dorsal region 7. In case a single piece of wire cannot be used for the radial, palmar and ulnar wire sections 3, 4, 5, then in an alternative embodiment the radial, palmar and ulnar wire sections 3, 4, 5 may be connected in welded fashion to obtain a continuous connection between the various sections 3, 4, 5.

In advantageous embodiments, the wire used for the wire frame 2 may have a substantially flat cross section free from sharp edges, wherein flatness of the wire allows for a larger contact area to prevent painful pressure points. In a further advantageous embodiment, all wire sections of the wrist orthosis 1 are made of substantially flat oval shaped wire.

In an exemplary embodiment, the wire frame 2 comprises silver, gold, bronze or titanium or a combination thereof as these materials are biologically inert and as such minimize bacterial growth on and around the wrist orthosis 1.

In advantageous embodiments, the wire frame 2 may be made of 92,5% silver (purity 99.999) and 7,5% copper (purity 99.999), sometimes referred to as 925/1000 silver.

In advantageous embodiments, the wire frame 2 and in particular the radial wire section 3, the palmar wire section 4, and the ulnar wire section 5 may each have a width between of 4 mm and 8 mm, preferably between 5 mm and 7mm. In an exemplary embodiment, the wire frame 2 and in particular the radial wire section 3, the palmar wire section 4, and the ulnar wire section 5 may each have a width of about 6 mm. These widths of the various wire sections are sufficient for providing distributed support/engagement to the skin such that pressure points are prevented.

In further advantageous embodiments, the wire frame 2 and in particular the radial wire section 3, the palmar wire section 4, and the ulnar wire section 5 may each have a thickness between 1 mm and 3 mm. In an exemplary embodiment, the wire frame 2 and in particular the radial wire section 3, the palmar wire section 4, and the ulnar wire section 5 may each have a thickness of about 2 mm.

Using the aforementioned wire specifications, such as the 925/1000 silver and/or the widths (4mm-8mm, 5mm-7mm, 6mm) and thicknesses (1mm-3mm, 2mm) of the wire sections allows for a sleek and low profile wire frame 2 that provides sufficient mechanical rigidity for orthopaedic support yet allow the wire frame 2 to be shaped/bent for achieving excellent fit of the wrist orthosis 1. It is further noted that the various wire sections may share the same width and thickness but they may also have different widths, for example.

As further depicted in Figure 2, in an exemplary embodiment, the radial wire section 3 comprises an inward bend 8 configured to extend along a first dorsal interosseous region 9 of the hand and wherein the ulnar wire section 5 comprises an inward bend 10 configured to extend along a dorsal ulnar region 11 of the hand. Here, the dorsal ulnar region 11 may be associated of being approximately opposite to a region of the abductor digiti mini on the volar side of the hand. Also, the inward bends 8, 10 must be understood as being convex as seen from the intermediate dorsal region 7. Both bends 8, 10 in this exemplary embodiment provide for a smooth ergonomic widening/increase of the separation distance O in the distal direction DD between the radial wire section 3 and the ulnar wire section 5 along the intermediate dorsal region 7. This smooth widening increases freedom for volar and dorsal finger movement whilst ensuring lateral stability, i.e. in ulnar direction and radial direction.

In an embodiment, the wire frame 2 further comprises a laterally extending wrist wire bridge 12 connecting the radial wire section 3 and ulnar wire section 5, wherein the wrist wire bridge 12 is configured to engage a dorsal side of the wrist. The wrist wire bridge 12 in this embodiment may be made of the same wire material as the radial wire section 3 and ulnar wire section 5. The wrist wire bridge 12 maintains the radial wire section 3 and ulnar wire section 5 at a fixed lateral separation distance to increase structural rigidity of the wrist orthosis 1 while maintaining the space between the radial wire section 3 and ulnar wire section 5 along the intermediate dorsal region 7. The wrist wire bridge 12 also provides dorsal support on the forearm for the release of pressure.

Note that the wrist wire bridge 12 still allows adjustments to be made to the separation distance O by e.g. bending the radial wire section 3 and ulnar wire section 5 toward or away from each other as explained earlier.

Further structural rigidity of the wire frame 2 can be achieved by an embodiment wherein the wire frame 2 further comprises a laterally extending first forearm wire bridge 13 connecting the radial wire section 3 and ulnar wire section 5 at each proximal end 3a, 5a thereof. That is, in this embodiment both the radial wire section 3 and ulnar wire section 5 are configured to terminate in the proximal direction PD at the depicted proximal ends 3a, 5a along the forearm. The first forearm wire bridge 13 laterally connects these proximal ends 3a, 5a and is configured to engage a dorsal side (or dorsal-radial side) of the forearm, thereby preventing expansion of the proximal ends 3a, 5a when the forearm engages the radial wire section 3 and ulnar wire section 5.

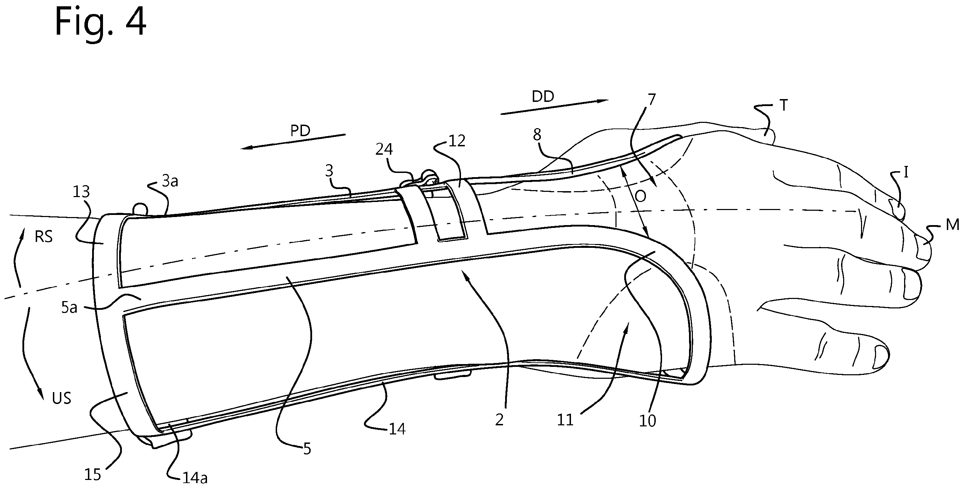

Turning to Figure 4, a dorsal-ulnar view of a wrist orthosis 1 is depicted according to an embodiment of the present invention. In the embodiment shown, the wire frame 2 comprises a further ulnar wire section 14, which connects to the ulnar or palmar wire section 5, 4 at the ulnar side of the hand. In particular, depending on the exact fit of the wrist orthosis 1, the further ulnar wire section 14 may be configured to connect to the ulnar or palmar wire section 5, 4 approximately at the region associated with the abductor digiti minimi of the hand, e.g. between the inward bend 10 and palmar wire section 4. The further ulnar wire section 14 then extends from the ulnar side of the hand in proximal direction, along the ulnar, toward and along the wrist and forearm substantially parallel to the ulnar wire section 5. As shown, the further ulnar wire section 14 is laterally spaced apart from the ulnar wire section 5. The further ulnar wire section 14 provides additional stability and fixation of the wrist orthosis and allows for further accurate placement of the wrist orthosis 1.

As shown in Figure 3, the further ulnar wire section 14 may be used to facilitate securing the wrist orthosis 1 to a patient. For example, in the embodiment shown, the wire frame 2 may further comprise a strap member 23 for releasably attaching the wire frame 2 to the forearm, so wherein the strap member 23 is releasably connected to the radial wire section 3 and the further ulnar wire section 14 and configured to laterally extend along a volar side of the forearm. In a further embodiment, the strap member 23 may comprise a separate distal strap portion 23a and a separate proximal strap portion 23b that are spaced apart, in proximal/distal direction, and adapted to laterally extend along a volar side of the forearm. This embodiment would further facilitate a comfortable and stable attachment of the wrist orthosis 1 to the patient. Note that in an embodiment, the distal strap portion 23a may be attachable to the radial wire section 3 at the wrist wire bridge 12 and wherein the proximal strap portion 23b is attachable to the radial wire section 3 near the proximal end 3a thereof.

Generally, the strap member 23 may be releasably attached to the wrist orthosis 1 by one or more strap hooks 24. In a further embodiment, depending on orthopaedic requirements, each of the distal and proximal strap portions 23a, 23b may be releasably attached to the wrist orthoses 1, e.g. to the radial wire section 3 and the ulnar wire section 5 or the further ulnar wire section 14 if it is used. In an advantageous embodiment, the one or more strap hooks 24 may made of the same material as the wire frame 2, such as silver, gold, titanium or any combination thereof.

Advantageously, the one or more strap hooks 24 and the wire frame 2 with various wire sections 3, 4, 5, 12, 13, 14, 15 may benefit from being made of silver, gold, bronze, titanium or a combination thereof, where these materials are biologically inert and as such minimize bacterial growth on and around the wrist orthosis 1.

In case the further ulnar wire section 14 is not used because of e.g. orthopaedic considerations, then it is of course possible to have an embodiment wherein the wire frame 2 comprises a strap member 23 for releasably attaching the wire frame 2 to the forearm and wherein the strap member 23 releasably connects to the radial wire section 3 and the ulnar wire section 5 and configured to laterally extend along a volar side of the forearm.

In Figure 4 it is further shown that in an embodiment the wire frame 2 may further comprise a second forearm wire bridge 15 that laterally connects the further ulnar wire section 14 and the ulnar wire section 5 at each proximal end 5a, 14a thereof, and wherein the second forearm wire bridge 15 is arranged to engage an ulnar side (or dorsal-ulnar side) of the forearm. In this embodiment the second forearm wire bridge 15 provides lateral stability and increases the structural rigidity by affixing the proximal end 14a of the further ulnar wire section 14 and the proximal end 5a of the ulnar wire section 5 to the latterly arranged second forearm wire bridge 15. The second forearm wire bridge 15 also ensures that the ulnar wire section 5 and the further ulnar wire section 14 are maintained substantially parallel to one another and at a desired lateral distance.

It is noted that in an advantageous embodiment the wrist wire bridge 12, the first forearm wire bridge 13 and the second forearm wire bridge 15 may each have the same wire specifications as mentioned earlier, e.g. being made of 92,5% silver (purity 99.999) and 7,5% copper (purity 99.999), also referred as 925/1000 silver.

In advantageous embodiments, the wrist wire bridge 12, the first forearm wire bridge 13, and the second forearm wire bridge 15 may each have a width between of 4 mm and 8 mm, preferably between 5 mm and 7mm. In an exemplary embodiment, the wrist wire bridge 12, the first forearm wire bridge 13, and the second forearm wire bridge 15 may each have a width about 6 mm.

In further advantageous embodiments, the wrist wire bridge 12, the first forearm wire bridge 13, and the second forearm wire bridge 15 may each have a thickness between 1 mm and 3 mm. In an exemplary embodiment, the wrist wire bridge 12, the first forearm wire bridge 13, and the second forearm wire bridge 15 may each have a thickness of about 2 mm.

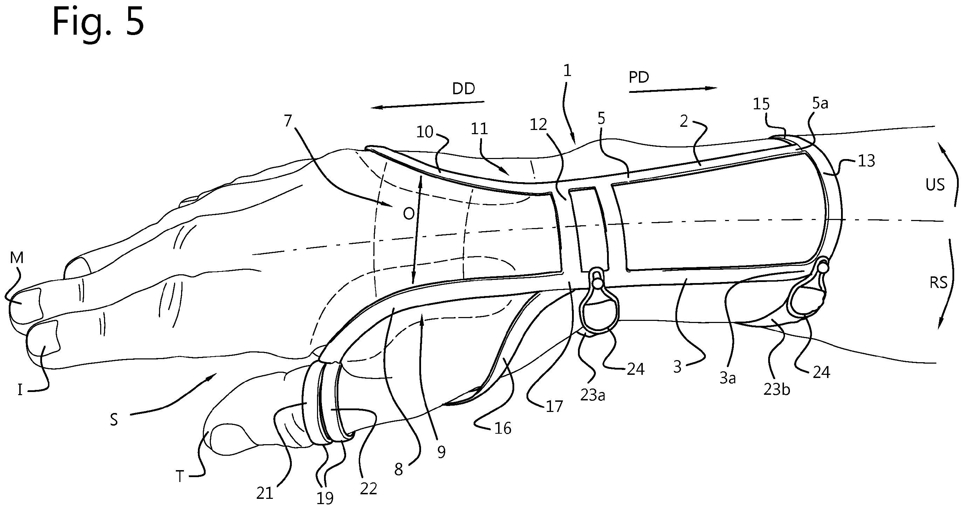

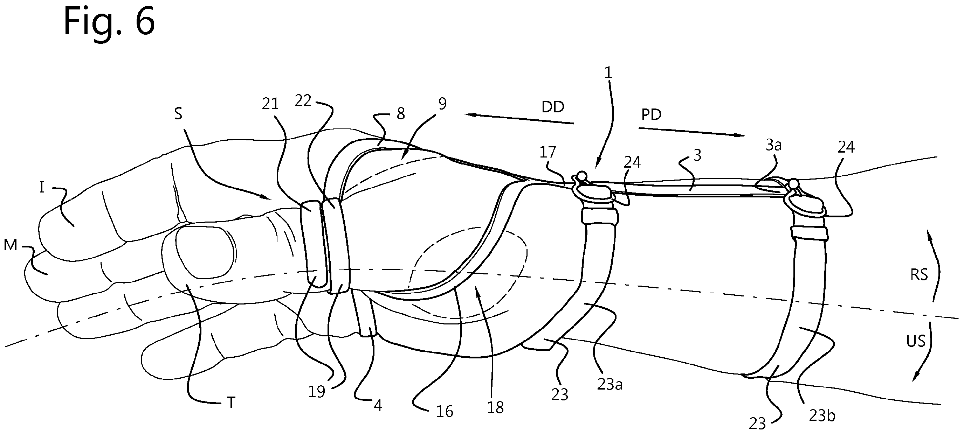

The wrist orthosis 1 of the present invention may be further adapted to provide further orthopaedic needs. For example, in addition to providing wrist support, the wrist orthosis 1 may be adapted to also provide thumb support. Such applications are exemplified in Figures 5 and 6, showing a dorsal view and volar-radial view, respectively, of a wrist orthosis 1 comprising a thumb brace according to an embodiment of the present invention.

In the embodiments shown, the wire frame 2 further comprises a thumb wire section 16 which is connected to the radial and palmar wire section 3, 4 at the interdigital space S and to a wrist engaging portion 17 of the radial wire section 3. The thumb wire section 16 is configured to extend, e.g. in loop wise fashion, along a thenar eminence region 18 of the hand. The thumb wire section 16 may be used, for example, to support the first metacarpal bone and first carpometacarpal joint. Note that in an advantageous embodiment the thumb wire section 16 connects to the wrist engaging portion 17 at the wrist wire bridge 12, thereby providing for improved rigidity and stability of the thumb wire section 16.

If additional thumb support is needed, then an embodiment may be considered wherein the wire frame 2 further comprises a ring shaped wire section 19 that connects to the radial and palmar wire section 3, 4, i.e. approximately where they meet, at the interdigital space S. The ring shaped wire section 19 is then configured to receive the thumb, e.g. a proximal phalanx part of a thumb. In this embodiment the ring shaped wire section 19 may be used to provide support to the first metacarpophalangeal joint (MP).

The ring shaped wire section 19 may be adapted in various ways to improve orthopaedic needs. For example, in an embodiment, the ring shaped wire section 19 may comprises two substantially parallel rings 21, 22 spaced apart at least along a portion of their circumference. This embodiment allows the two parallel rings 21, 22 to be adapted separately if needed to accommodate a particular anatomy for e.g. avoiding discomfort to a patient yet provide the desired support of the thumb. Another advantage of this embodiment is that a width of the ring shaped wire section 19 can be enlarged to improve thumb support by using the same type of wire as used for e.g. the radial, palmar and ulnar wire sections 3, 4, 5. A further embodiment is possible wherein the ring shaped wire section 19 is shaped as a spiral comprising at least one and a half turns, so that a single piece of wire can be used to shape the ring shaped wire section 19 and provide sufficient width to the ring shaped wire section 19 to minimize pressure points and comfortably support a patient's thumb.

Note that like the other wire sections, the ring shaped wire section 19 may be made of 925/1000 silver and/or the widths and thicknesses as specified earlier.

The present invention embodiments have been described above with reference to a number of exemplary embodiments as shown in and described with reference to the drawings. Modifications and alternative implementations of some parts or elements are possible as long as they are included in the scope of protection as defined in the appended claims.

## Claims

1. A wrist orthosis (1) for support of a patient's wrist, comprising a wire frame (2) which is configured to a engage a forearm (F), wrist (W), and hand (H) of a patient, wherein the wire frame (2) comprises a continuous connection of
a radial wire section (3) configured to extend in a distal direction along the forearm, wrist and a radial side of the hand to an interdigital space (S) between the thumb and index finger of the hand, wherein the radial wire section (3) is connected to
a palmar wire section (4) configured to extend in a lateral direction from the interdigital space (S) along a palmar region (6) of the hand toward an ulnar side of the hand, and wherein the palmar wire section (4) is connected to an
ulnar wire section (5) configured to extend from the ulnar side of the hand in proximal direction toward and along the wrist and forearm; and wherein
the radial wire section (3) and the ulnar wire section (5) are laterally spaced apart along an intermediate dorsal region (7) of the hand.

2. The wrist orthosis (1) according to claim 1, wherein the radial wire section (3) comprises an inward bend (8) configured to extend along a first dorsal interosseous region (9) of the hand and wherein the ulnar wire section (5) comprises an inward bend (10) configured to extend along a dorsal ulnar region (11) of the hand.

3. The wrist orthosis (1) according to claim 1 or 2, wherein the radial wire section (3), the palmar wire section (4), and the ulnar wire section (5) are formed as a continuous single piece of bent wire.

4. The wrist orthosis (1) according to any of claims 1-3, wherein the wire frame (2) further comprises a laterally extending wrist wire bridge (12) connecting the radial wire section (3) and ulnar wire section (5), wherein the wrist wire bridge (12) is configured to engage a dorsal side of the wrist.

5. The wrist orthosis (1) according to any of claims 1-4, wherein the wire frame (2) further comprises a laterally extending first forearm wire bridge (13) connecting the radial wire section (3) and ulnar wire section (5) at each proximal end (3a, 5a) thereof, wherein the first forearm wire bridge (13) is configured to engage a dorsal side of the forearm.

6. The wrist orthosis (1) according to any of claims 1-5, where the wire frame (2) further comprises a further ulnar wire section (14) connected to the ulnar or palmar wire section (3, 4) at the ulnar side of the hand, and wherein the further ulnar wire section (14) is configured to extend from the ulnar side of the hand in proximal direction toward and along the wrist and forearm substantially parallel to the ulnar wire section (5) and spaced apart therefrom.

7. The wrist orthosis (1) according to claim 6, wherein the wire frame (2) further comprises a second forearm wire bridge (15) laterally connecting the further ulnar wire section (14) and the ulnar wire section (5) at each proximal end (5a, 14a) thereof, wherein the second forearm wire bridge (15) is arranged to engage an ulnar side of the forearm.

8. The wrist orthosis (1) according to any of claims 1-7, wherein the wire frame (2) further comprises a thumb wire section (16) connected to the radial or palmar wire section (3, 4) at the interdigital space (S) and to a wrist engaging portion (17) of the radial wire section (3), and wherein the thumb wire section (16) is configured to extend along a thenar eminence region (18) of the hand.

9. The wrist orthosis (1) according to any of claims 1-8, wherein the wire frame (2) further comprises a ring shaped wire section (19) connected to the radial or palmar wire section (3, 4) at the interdigital space (S), the ring shaped wire section (19) being configured to receive a proximal phalanx part of a thumb.

10. The wrist orthosis (1) according to claim 9, wherein the ring shaped wire section (19) comprises two substantially parallel rings (21, 22) spaced apart at least along a portion of their circumference.

11. The wrist orthosis (1) according to claim 9, wherein the ring shaped wire section (19) is shaped as a spiral comprising at least one and a half turns.

12. The wrist orthosis (1) according to any of claims 1-10, wherein the wire frame (2) further comprises a strap member (23) for releasably attaching the wire frame (2) to the forearm,
wherein the strap member (23) is releasably connected to the radial wire section (3) and the ulnar wire section (5) and configured to laterally extend along a volar side of the forearm.

13. The wrist orthosis (1) according to claim 6 or 7, wherein the wire frame (2) further comprises a strap member (23) for releasably attaching the wire frame (2) to the forearm,
wherein the strap member (23) is releasably connected to the radial wire section (3) and the further ulnar wire section (14) and configured to laterally extend along a volar side of the forearm.

14. The wrist orthosis (1) according to any of claims 1-13 , wherein the wire frame (2) is a metallic or a metal alloy wire frame.

15. The wrist orthosis (1) according to claim 14, wherein the wire frame (2) comprises silver, gold, bronze or titanium or a combination thereof.

## Patentansprüche

1. Eine Handgelenk Orthese (1) für die Unterstützung des Handgelenks eines Patienten, aufweisend ein Drahtgestell (2), welches konfiguriert ist, um in Eingriff zu nehmen einen Unterarm (F), ein Handgelenk (W) und eine Hand (H) eines Patienten, wobei das Drahtgestell (2) aufweist eine kontinuierliche Verbindung von
einem radialen Drahtabschnitt, konfiguriert, um sich zu erstrecken in eine distale Richtung entlang des Unterarms, des Handgelenks und einer radialen Seite der Hand hin zu einem zwischen Finger Raum (S) zwischen dem Daumen und dem Zeigefinger der Hand, wobei der radiale Drahtabschnitt (3) verbunden ist mit
einem hohlhandseitigen Drahtabschnitt (4), konfiguriert, um sich zu erstrecken in eine laterale Richtung von dem zwischen Finger Raum (S) entlang einer hohlhandseitigen Region (6) der Hand hin zu einer Ellen Seite der Hand und wobei der hohlhandseitige Drahtabschnitt (4) verbunden ist zu einem
Ellen Drahtabschnitt (5), konfiguriert, um sich zu erstrecken von der Ellen Seite der Hand in proximaler Richtung hin zu und entlang des Handgelenks und Unterarms; und wobei
der radiale Drahtabschnitt (3) und der Ellen Drahtabschnitt(5) lateral beabstandet sind entlang einer intermediären dorsalen Region (7) der Hand.

2. Die Handgelenk Orthese (1) gemäß Anspruch 1, wobei der radiale Drahtabschnitt (3) aufweist eine einwärts Biegung (8), konfiguriert, um sich zu erstrecken entlang einer ersten zwischen Knochen Region (9) der Hand und wobei der Ellen Drahtabschnitt (5) aufweist eine einwärts Biegung (10), konfiguriert, um sich zu erstrecken entlang einer dorsalen Ellen Region (11) der Hand.

3. Die Handgelenk Orthese (1) gemäß Anspruch 1 oder 2, wobei der radiale Drahtabschnitt (3), der hohlhandseitige Drahtabschnitt (4) und der Ellen Drahtabschnitt (5) ausgebildet sind als kontinuierliches, einzelnes Stück gebogener Draht.

4. Die Handgelenk Orthese (1) gemäß irgendeinem der Ansprüche 1 bis 3, wobei das Drahtgestell (2) ferner aufweist eine sich lateral erstreckende Handgelenk Drahtbrücke (12), welche verbindet den radialen Drahtabschnitt (3) und den Ellen Drahtabschnitt (5), wobei die Handgelenk Drahtbrücke (12) konfiguriert ist, um in Eingriff zu nehmen eine dorsale Seite des Handgelenks.

5. Die Handgelenk Orthese (1) gemäß irgendeinem der Ansprüche 1 bis 4, wobei das Drahtgestell (2) ferner aufweist eine sich lateral erstreckende erste Unterarm Drahtbrücke (13), welche verbindet den radialen Drahtabschnitt (3) und dien Ellen Drahtabschnitt (5) an jedem proximalen Ende (3a, 5a) davon, wobei die erste Unterarm Drahtbrücke (13) konfiguriert ist, um in Eingriff zu nehmen eine dorsale Seite des Unterarms.

6. Die Handgelenk Orthese (1) gemäß irgendeinem der Ansprüche 1 bis 5, wobei das Drahtgestell (2) ferner aufweist einen weiteren Ellen Drahtabschnitt (14), verbunden mit dem Ellen oder hohlhandseitigen Drahtabschnitt (3, 4) an der Ellen Seite der Hand, und wobei der weitere Ellen Drahtabschnitt (14) konfiguriert ist, sich zu erstrecken von der Ellen Seite der Hand in proximaler Richtung hin zu und entlang des Handgelenks und Unterarms im Wesentlichen parallel zu dem Ellen Drahtabschnitt (5) und beabstandet davon.

7. Die Handgelenk Orthese (1) gemäß Anspruch 6, wobei das Drahtgestell (2) ferner aufweist eine zweite Unterarm Drahtbrücke (15), welche lateral verbindet den weiteren Ellen Drahtabschnitt (14) und den Ellen Drahtabschnitt (5) an jedem proximalen Ende (5a, 14a) davon, wobei die zweite Unterarm Drahtbrücke (15) angeordnet ist, um in Eingriff zu nehmen eine Ellen Seite des Unterarms.

8. Die Handgelenk Orthese (1) gemäß irgendeinem der Ansprüche 1 bis 7, wobei das Drahtgestell (2) ferner aufweist einen Daumen Drahtabschnitt (16), verbunden mit dem radialen oder hohlhandseitigen Drahtabschnitt (3, 4) an dem zwischen Finger Raum (S) und mit einem Handgelenk Eingriff Teil (17) des radialen Drahtabschnitts (3) und wobei der Daumen Drahtabschnitt (16) konfiguriert ist, um sich zu erstrecken entlang einer Handballen Region (18) der Hand.

9. Die Handgelenk Orthese (1) gemäß irgendeinem der Ansprüche 1 bis 8, wobei das Drahtgestell (2) ferner aufweist einen ringförmigen Drahtabschnitt (19), verbunden mit dem radialen oder hohlhandseitigen Drahtabschnitt (3, 4) an dem zwischen Finger Raum (S), wobei der ringförmige Drahtabschnitt (19) konfiguriert ist, um aufzunehmen einen proximalen Fingerglied Teil eines Daumens.

10. Die Handgelenk Orthese (1) gemäß Anspruch 9, wobei der ringförmige Drahtabschnitt (19) aufweist zwei im Wesentlichen parallele Ringe (21, 22), beabstandet zumindest entlang eines Teils von deren Umfang.

11. Die Handgelenk Orthese (1) gemäß Anspruch 9, wobei der ringförmige Drahtabschnitt (19) geformt ist als eine Spirale aufweisend zumindest eineinhalb Drehungen.

12. Die Handgelenk Orthese (1) gemäß irgendeinem der Ansprüche 1 bis 10, wobei das Drahtgestell (2) ferner aufweist ein Gurtelement (23) zum lösbaren Befestigen des Drahtgestells (2) an den Unterarm,
wobei das Gurtelement (23) lösbar verbunden ist mit dem radialen Drahtabschnitt (3) und dem Ellen Drahtabschnitt (5) und konfiguriert ist, um sich lateral zu erstrecken entlang einer volaren Seite des Unterarms.

13. Die Handgelenk Orthese (1) gemäß Anspruch 6 oder 7, wobei das Drahtgestell (2) ferner aufweist ein Gurtelement (23) zum lösbaren Befestigen des Drahtgestells (2) an den Unterarm,
wobei das Gurtelement (23) lösbar verbunden ist mit dem radialen Drahtabschnitt (3) und dem weiteren Ellen Drahtabschnitt (14) und konfiguriert ist, um sich lateral zu erstrecken entlang einer volaren Seite des Unterarms.

14. Die Handgelenk Orthese (1) gemäß irgendeinem der Ansprüche 1 bis 13, wobei das Drahtgestell (2) ein Metall oder Metall-Legierung Drahtgestell ist.

15. Die Handgelenk Orthese (1) gemäß Anspruch 14, wobei das Drahtgestell (2) aufweist Silber, Gold, Bronze oder Titan oder eine Kombination davon.

## Revendications

1. Orthèse de poignet (1) pour le support du poignet d'un patient, comprenant une armature en fil métallique (2) qui est configurée pour venir en prise avec un avant-bras (F), un poignet (W) et une main (H) d'un patient, dans laquelle l'armature en fil métallique (2) comprend une connexion continue
d'une section de fil métallique radiale (3) configurée pour s'étendre dans une direction distale le long de l'avant-bras, du poignet et d'un côté radial de la main jusqu'à un espace interdigital (S) entre le pouce et l'index de la main, dans laquelle la section de fil métallique radiale (3) est connecté à
une section de fil métallique palmaire (4) configurée pour s'étendre dans une direction latérale depuis l'espace interdigital (S) le long d'une région palmaire (6) de la main vers un côté ulnaire de la main, et dans laquelle la section de fil métallique palmaire (4) est connectée à
une section de fil métallique ulnaire (5) configurée pour s'étendre depuis le côté ulnaire de la main dans une direction proximale vers et le long du poignet et de l'avant-bras ; et dans laquelle
la section de fil métallique radiale (3) et la section de fil métallique ulnaire (5) sont espacées latéralement le long d'une région dorsale intermédiaire (7) de la main.

2. Orthèse de poignet (1) selon la revendication 1, dans laquelle la section de fil métallique radiale (3) comprend un coude vers l'intérieur (8) configuré pour s'étendre le long d'une première région interosseuse dorsale (9) de la main et dans laquelle la section de fil métallique ulnaire (5) comprend un coude vers l'intérieur (10) configuré pour s'étendre le long d'une région ulnaire dorsale (11) de la main.

3. Orthèse de poignet (1) selon la revendication 1 ou 2, dans laquelle la section de fil métallique radiale (3), la section de fil métallique palmaire (4) et la section de fil métallique ulnaire (5) sont formées sous la forme d'une pièce continue unique de fil métallique coudé.

4. Orthèse de poignet (1) selon l'une quelconque des revendications 1 à 3, dans laquelle l'armature en fil métallique (2) comprend en outre un pont de fil métallique de poignet s'étendant latéralement (12) reliant la section de fil métallique radiale (3) et la section de fil métallique ulnaire (5), dans laquelle le pont de fil métallique de poignet (12) est configuré pour venir en prise avec un côté dorsal du poignet.

5. Orthèse de poignet (1) selon l'une quelconque des revendications 1 à 4, dans laquelle l'armature en fil métallique (2) comprend en outre un premier pont de fil métallique d'avant-bras s'étendant latéralement (13) reliant la section de fil métallique radiale (3) et la section de fil métallique ulnaire (5) à chaque extrémité proximale (3a, 5a) de celui-ci, dans laquelle le premier pont de fil métallique d'avant-bras (13) est configuré pour venir en prise avec un côté dorsal de l'avant-bras.

6. Orthèse de poignet (1) selon l'une quelconque des revendications 1 à 5, dans laquelle l'armature en fil métallique (2) comprend en outre une section de fil métallique ulnaire supplémentaire (14) reliée à la section de fil métallique ulnaire ou palmaire (3, 4) au niveau du côté ulnaire de la main, et dans laquelle la section de fil métallique ulnaire supplémentaire (14) est configurée pour s'étendre depuis le côté ulnaire de la main dans une direction proximale vers et le long du poignet et de l'avant-bras sensiblement parallèlement à la section de fil métallique ulnaire (5) et espacée de celle-ci.

7. Orthèse de poignet (1) selon la revendication 6, dans laquelle l'armature en fil métallique (2) comprend en outre un second pont de fil métallique d'avant-bras (15) reliant latéralement la section de fil métallique ulnaire supplémentaire (14) et la section de fil métallique ulnaire (5) à chaque extrémité proximale (5a, 14a) de celui-ci, dans laquelle le second pont de fil métallique d'avant-bras (15) est agencé pour venir en prise avec un côté ulnaire de l'avant-bras.

8. Orthèse de poignet (1) selon l'une quelconque des revendications 1 à 7, dans laquelle l'armature en fil métallique (2) comprend en outre une section de fil métallique de pouce (16) reliée à la section de fil métallique radiale ou palmaire (3, 4) au niveau de l'espace interdigital (S) et à une partie de mise en prise de poignet (17) de la section de fil métallique radiale (3), et dans laquelle la section de fil métallique de pouce (16) est configurée pour s'étendre le long d'une région d'éminence thénar (18) de la main.

9. Orthèse de poignet (1) selon l'une quelconque des revendications 1 à 8, dans laquelle l'armature en fil métallique (2) comprend en outre une section de fil métallique en forme d'anneau (19) reliée à la section de fil métallique radiale ou palmaire (3, 4) au niveau de l'espace interdigital (S), la section de fil métallique en forme d'anneau (19) étant configurée pour recevoir une partie de phalange proximale d'un pouce.

10. Orthèse de poignet (1) selon la revendication 9, dans laquelle la section de fil métallique en forme d'anneau (19) comprend deux anneaux sensiblement parallèles (21, 22) espacés au moins le long d'une partie de leur circonférence.

11. Orthèse de poignet (1) selon la revendication 9, dans laquelle la section de fil métallique en forme d'anneau (19) est réalisée sous la forme d'une spirale comprenant au moins un tour et demi.

12. Orthèse de poignet (1) selon l'une quelconque des revendications 1 à 10, dans laquelle l'armature en fil métallique (2) comprend en outre un élément de sangle (23) pour attacher de manière amovible l'armature en fil métallique (2) à l'avant-bras,
dans laquelle l'élément de sangle (23) est connecté de manière amovible à la section de fil métallique radiale (3) et à la section de fil métallique ulnaire (5) et configuré pour s'étendre latéralement le long d'un côté palmaire de l'avant-bras.

13. Orthèse de poignet (1) selon la revendication 6 ou 7, dans laquelle l'armature en fil métallique (2) comprend en outre un élément de sangle (23) pour attacher de manière amovible l'armature en fil métallique (2) à l'avant-bras,
dans laquelle l'élément de sangle (23) est connecté de manière amovible à la section de fil métallique radial (3) et à la section de fil métallique ulnaire supplémentaire (14) et configuré pour s'étendre latéralement le long d'un côté palmaire de l'avant-bras.

14. Orthèse de poignet (1) selon l'une quelconque des revendications 1 à 13, dans laquelle l'armature en fil métallique (2) est une armature en fil métallique ou d'alliage métallique.

15. Orthèse de poignet (1) selon la revendication 14, dans laquelle l'armature en fil métallique (2) comprend de l'argent, de l'or, du bronze ou du titane ou une combinaison de ceux-ci.
